# EUROPEAN PATENT SPECIFICATION

(11) **EP 3 843 835 B1**
(45) Date of publication and mention of the grant of the patent: **06.05.2026**
(21) Application number: 19854262.3
(22) Date of filing: 23.08.2019
(51) Int. Cl.: A61N 1/375, A61N 1/05, A61N 1/36

(54) **SYSTEM UTILIZING AN OFFSET EXTERNAL MAGNET**
SYSTEM UNTER VERWENDUNG EINES EXTERNEN VERSATZMAGNETEN
SYSTÈME UTILISANT UN AIMANT EXTERNE DÉCALÉ

(30) Priority: 27.08.2018 US 201862723222 P
(43) Date of publication of application: 07.07.2021
(73) Proprietor: Cochlear Limited, Macquarie University, New South Wales 2109 (AU)
(72) Inventor: FUNG, Wilson, Macquarie University, New South Wales 2109 (AU)
(74) Representative: Reddie & Grose LLP
(86) International application number: PCT/IB2019/057124
(87) International publication number: WO 2020/044190

(56) References cited:
- WO-A1-2017/027045
- KR-B1- 101 465 001
- US-A1- 2004 260 362
- US-A1- 2010 004 716
- US-A1- 2012 229 236
- US-A1- 2017 361 117
- US-B1- 6 246 911

## Description

### BACKGROUND

### Field

The present application relates generally to implantable auditory prostheses, and more specifically systems and methods using magnetic force to transcutaneously mechanically couple an external portion of an auditory prosthesis with an implanted portion of the auditory prosthesis.

### Description of the Related Art

Hearing loss, which may be due to many different causes, is generally of two types, conductive and/or sensorineural. Conductive hearing loss occurs when the normal mechanical pathways of the outer and/or middle ear are impeded, for example, by damage to the ossicular chain or ear canal. Sensorineural hearing loss occurs when there is damage to the inner ear, or to the nerve pathways from the inner ear to the brain. Auditory prostheses of various types are widely used to improve the lives of users. Such devices include, for example, hearing aids, cochlear implants, bone conduction implants, middle ear implants, and electro-acoustic devices.

Individuals who suffer from conductive hearing loss typically have some form of residual hearing because the hair cells in the cochlea are undamaged. As a result, individuals suffering from conductive hearing loss might receive an auditory prosthesis that generates mechanical motion of the cochlea fluid instead of a hearing aid based on the type of conductive loss, amount of hearing loss and customer preference. Such prostheses include, for example, bone conduction devices and direct acoustic stimulators.

In many people who are profoundly deaf, however, the reason for their deafness is sensorineural hearing loss. Those suffering from some forms of sensorineural hearing loss are unable to derive suitable benefit from auditory prostheses that generate mechanical motion of the cochlea fluid. Such individuals can benefit from implantable auditory prostheses that stimulate nerve cells of the recipient's auditory system in other ways (e.g., electrical, optical, and the like). Cochlear implants are often proposed when the sensorineural hearing loss is due to the absence or destruction of the cochlea hair cells, which transduce acoustic signals into nerve impulses. Auditory brainstem stimulators might also be proposed when a recipient experiences sensorineural hearing loss due to damage to the auditory nerve.

US 6 246 911 B1 describes a hearing prothesis comprising a pair of coil assemblies that are intended to be offset or displaced laterally by a predetermined distance to reduce variations in the signal transmission between the internal and external coils. This offset is achieved by locating a magnet at an eccentric position relative to a circular transmitter coil, and fixing said magnet to the transmitter coil with arms. US 6 246 911 B1 also describes another arrangement whereby the magnet of the external component can be selectively moved between different positions relative to its transmitter coil. This is achieved by locating the magnet within an elongate capsule and moving the magnet within the space defined by the elongate capsule.

### SUMMARY

The invention is defined by the independent claim 1 to which reference is now made. Any methods disclosed hereinafter do not form part of the scope of the invention, and are mentioned for illustrative purposes only. In accordance with the invention, there is provided an apparatus which comprises a cavity and a ferromagnet assembly having a first centroidal axis. The ferromagnet assembly is configured to be contained within and removable from the cavity. The ferromagnet assembly comprises a first portion comprising at least one non-ferromagnetic material. The ferromagnet assembly further comprises a second portion comprising at least one ferromagnetic material, the second portion having a second centroidal axis that is offset from the first centroidal axis.

The present disclosure includes an apparatus which comprises a housing configured to be placed over a portion of skin of a recipient, the portion of skin overlaying an implanted device. The implanted device comprises an internal inductive communication coil and an internal ferromagnetic material. The apparatus further comprises an external inductive communication coil within the housing. The external inductive communication coil is configured to be in inductive communication with the internal inductive communication coil. The apparatus further comprises an external ferromagnet contained within a cavity of the housing and substantially filling the cavity. The external ferromagnet comprises a ferromagnetic portion and a non-ferromagnetic portion. The ferromagnetic portion is configured to be magnetically attracted to the internal ferromagnetic material. The cavity has a center that defines a line with a center of the external inductive communication coil and the ferromagnetic portion has a center that is offset from the line.

The present disclosure also includes a method which comprises providing a sound processor assembly of an auditory prosthesis. The assembly is configured to be placed over a portion of skin of a recipient overlaying an implanted portion of the auditory prosthesis. The sound processor assembly comprises at least one external inductive communication coil configured to be in inductive communication with at least one internal inductive communication coil of the implanted portion of the auditory prosthesis. The sound processor assembly further comprises a cavity configured to contain a ferromagnet assembly. The method further comprises, in response to a thickness of the portion of skin, selecting a ferromagnet assembly from two or more ferromagnet assemblies configured to be contained within the cavity. Each of the two or more ferromagnet assemblies is configured to, upon being contained within the cavity and the sound processor assembly being placed over the portion of skin, displace the at least one external inductive communication coil relative to the at least one internal inductive communication coil by a corresponding offset in a direction parallel to the portion of skin while providing sufficient magnetic force to retain the sound processor assembly over the portion of skin.

### BRIEF DESCRIPTION OF THE DRAWINGS

Embodiments are described herein in conjunction with the accompanying drawings, in which:
FIG. 1A is a perspective view of an example cochlear implant auditory prosthesis implanted in a recipient in accordance with certain embodiments described herein;
FIG. 1B schematically illustrates a cross-sectional view of an example external component and an example internal component of the example cochlear implant auditory prosthesis of FIG. 1A;
FIG. 2 illustrates the coupling coefficient k between at least one first inductive communication coil and at least one second inductive communication coil depending on the thickness of the recipient's skin;
FIGs. 3A-3C schematically illustrate an example apparatus in accordance with certain embodiments described herein;
FIG. 4A schematically illustrates an example apparatus utilized with a first skin flap thickness and FIGs. 4B-4D schematically illustrate an example apparatus utilized with different skin flap thicknesses in accordance with certain embodiments described herein; and
FIG. 5 is a flow diagram of an example method of the present disclosure.

### DETAILED DESCRIPTION

Certain embodiments described herein advantageously provide a variable offset, in a direction parallel to the recipient's skin, between the external coil of an external component of an auditory prosthesis and the implanted coil for transcutaneous communication. The amount of offset can be easily adjusted, in response at least in part to the recipient's skin flap thickness, by selecting and using a ferromagnet assembly that fits within the external component and that has a ferromagnetic portion that is offset from a center of the ferromagnet assembly. This offset and the concomitant reduction of the coupling coefficient k between the external and implanted coils, is achieved without any change to existing designs of the external component, the retention force provided by the ferromagnet assembly or the implant magnet.

The teachings detailed herein are applicable, in at least some embodiments, to any type of auditory prosthesis utilizing an implantable actuator assembly including but not limited to: electro-acoustic electrical/acoustic systems, cochlear implant devices, implantable hearing aid devices, middle ear implant devices, bone conduction devices (e.g., active bone conduction devices; passive bone conduction devices, percutaneous bone conduction devices; transcutaneous bone conduction devices), Direct Acoustic Cochlear Implant (DACI), middle ear transducer (MET), electro-acoustic implant devices, other types of auditory prosthesis devices, and/or combinations or variations thereof, or any other suitable hearing prosthesis system with or without one or more external components. Embodiments can include any type of auditory prosthesis that can utilize the teachings detailed herein and/or variations thereof. In some embodiments, the teachings detailed herein and/or variations thereof can be utilized in other types of prostheses beyond auditory prostheses. For example, the concepts described herein can be applied to any of a variety of implantable medical devices that utilize the transfer of power and/or data between an implanted component and an external component via inductive coupling (e.g., pacemakers; implantable EEG monitoring devices; visual prostheses).

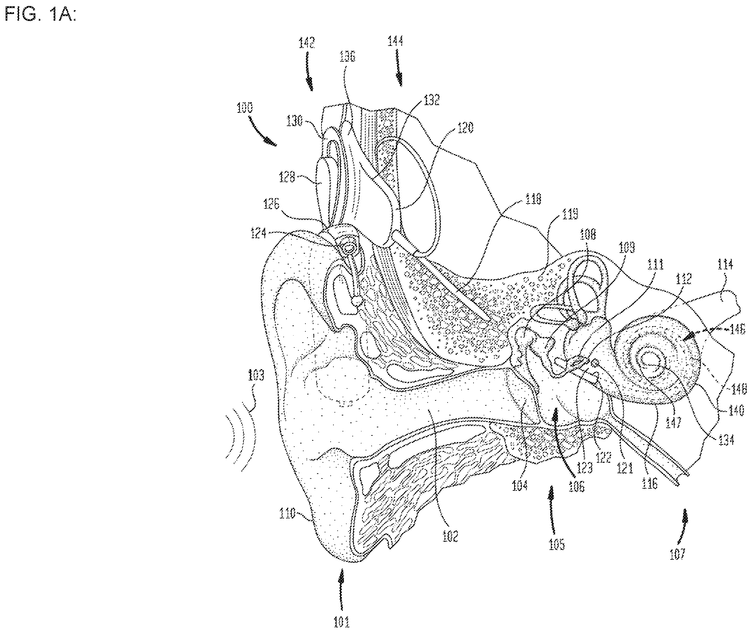

FIG. 1A is a perspective view of an example cochlear implant auditory prosthesis 100 implanted in a recipient in accordance with certain embodiments described herein. The example auditory prosthesis 100 is shown in FIG. 1A as comprising an implanted stimulator unit 120 (e.g., an actuator) and an external microphone assembly 124 (e.g., a partially implantable cochlear implant). An example auditory prosthesis 100 (e.g., a totally implantable cochlear implant) in accordance with certain embodiments described herein can replace the external microphone assembly 124 shown in FIG. 1A with a subcutaneously implantable assembly comprising an acoustic transducer (e.g., microphone), as described more fully herein.

As shown in FIG. 1A, the recipient has an outer ear 101, a middle ear 105, and an inner ear 107. In a fully functional ear, the outer ear 101 comprises an auricle 110 and an ear canal 102. An acoustic pressure or sound wave 103 is collected by the auricle 110 and is channeled into and through the ear canal 102. Disposed across the distal end of the ear canal 102 is a tympanic membrane 104 which vibrates in response to the sound wave 103. This vibration is coupled to oval window or fenestra ovalis 112 through three bones of middle ear 105, collectively referred to as the ossicles 106 and comprising the malleus 108, the incus 109, and the stapes 111. The bones 108, 109, and 111 of the middle ear 105 serve to filter and amplify the sound wave 103, causing the oval window 112 to articulate, or vibrate in response to vibration of the tympanic membrane 104. This vibration sets up waves of fluid motion of the perilymph within cochlea 140. Such fluid motion, in turn, activates tiny hair cells (not shown) inside the cochlea 140. Activation of the hair cells causes appropriate nerve impulses to be generated and transferred through the spiral ganglion cells (not shown) and auditory nerve 114 to the brain (also not shown) where they are perceived as sound.

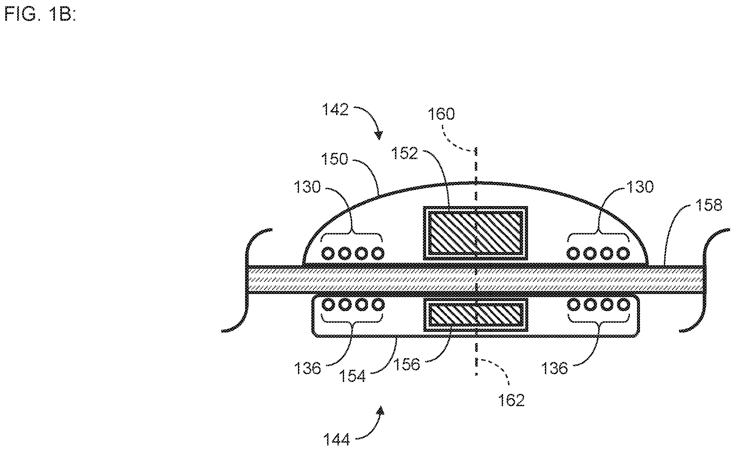

As shown in FIG. 1A, the example auditory prosthesis 100 comprises one or more components which are temporarily or permanently implanted in the recipient. The example auditory prosthesis 100 is shown in FIG. 1 with an external component 142 which is directly or indirectly attached to the recipient's body, and an internal component 144 which is temporarily or permanently implanted in the recipient (e.g., positioned in a recess of the temporal bone adjacent auricle 110 of the recipient). The external component 142 typically comprises one or more sound input elements (e.g., an external microphone 124) for detecting sound, a sound processing unit 126 (e.g., disposed in a Behind-The-Ear unit), a power source (not shown), and an external transmitter unit 128. In the illustrative embodiments of FIG. 1A, the external transmitter unit 128 comprises at least one first inductive communication coil 130 (e.g., a wire antenna coil comprising multiple turns of electrically insulated single-strand or multi-strand platinum or gold wire) and, preferably, a magnet (not shown) secured directly or indirectly to the at least one first inductive communication coil 130. The at least one first inductive communication coil 130 of the external transmitter unit 128 is part of an inductive radio frequency (RF) communication link with the internal component 144. The sound processing unit 126 processes the output of the microphone 124 that is positioned externally to the recipient's body, in the depicted embodiment, by the recipient's auricle 110. The sound processing unit 126 generates encoded signals, sometimes referred to herein as encoded data signals, which are provided to the external transmitter unit 128 (e.g., via a cable). As will be appreciated, the sound processing unit 126 can utilize digital processing techniques to provide frequency shaping, amplification, compression, and other signal conditioning, including conditioning based on recipient-specific fitting parameters.

The power source of the external component 142 is configured to provide power to the auditory prosthesis 100, where the auditory prosthesis 100 includes a battery (e.g., located in the internal component 144, or disposed in a separate implanted location) that is recharged by the power provided from the external component 142 (e.g., via a transcutaneous energy transfer link). The transcutaneous energy transfer link is used to transfer power and/or data to the internal component 144 of the auditory prosthesis 100. Various types of energy transfer, such as infrared (IR), electromagnetic, capacitive, and inductive transfer, may be used to transfer the power and/or data from the external component 142 to the internal component 144. During operation of the auditory prosthesis 100, the power stored by the rechargeable battery is distributed to the various other implanted components as needed.

The internal component 144 comprises an internal receiver unit 132, a stimulator unit 120, and an elongate electrode assembly 118. In some embodiments, the internal receiver unit 132 and the stimulator unit 120 are hermetically sealed within a biocompatible housing. The internal receiver unit 132 comprises at least one second inductive communication coil 136 (e.g., a wire antenna coil comprising multiple turns of electrically insulated single-strand or multi-strand platinum or gold wire), and preferably, a magnet (also not shown) fixed relative to the at least one second inductive communication coil 136. The internal receiver unit 132 and the stimulator unit 120 are hermetically sealed within a biocompatible housing, sometimes collectively referred to as a stimulator/receiver unit. The at least one second inductive communication coil 136 receives power and/or data signals from the at least one first inductive communication coil 130 via a transcutaneous energy transfer link (e.g., an inductive RF link). The stimulator unit 120 generates electrical stimulation signals based on the data signals, and the stimulation signals are delivered to the recipient via the elongate electrode assembly 118.

The elongate electrode assembly 118 has a proximal end connected to the stimulator unit 120, and a distal end implanted in the cochlea 140. The electrode assembly 118 extends from the stimulator unit 120 to the cochlea 140 through the mastoid bone 119. In some embodiments, the electrode assembly 118 may be implanted at least in the basal region 116, and sometimes further. For example, the electrode assembly 118 may extend towards apical end of cochlea 140, referred to as cochlea apex 134. In certain circumstances, the electrode assembly 118 may be inserted into the cochlea 140 via a cochleostomy 122. In other circumstances, a cochleostomy may be formed through the round window 121, the oval window 112, the promontory 123, or through an apical turn 147 of the cochlea 140.

The elongate electrode assembly 118 comprises a longitudinally aligned and distally extending array 146 of electrodes or contacts 148, sometimes referred to as electrode or contact array 146 herein, disposed along a length thereof. Although the electrode array 146 can be disposed on the electrode assembly 118, in most practical applications, the electrode array 146 is integrated into the electrode assembly 118 (e.g., the electrode array 146 is disposed in the electrode assembly 118). As noted, the stimulator unit 120 generates stimulation signals which are applied by the electrodes 148 to the cochlea 140, thereby stimulating the auditory nerve 114.

FIG. 1B schematically illustrates a cross-sectional view of an example conventional arrangement of an external component 142 and an example internal component 144 of the example cochlear implant auditory prosthesis 100 of FIG. 1A. Referring to FIG. 1A, the example external component 142 comprises a housing 150 containing at least one microphone 124, a sound processing unit 126, a power source, and a transmitter unit 128. The example external component 142 further comprises a first ferromagnetic portion 152 (e.g., a first magnet) contained within the housing 150. The example internal component 144 comprises a biocompatible housing 154 which contains (e.g., within a hermetically sealed region) an implanted receiver unit 132, a stimulator unit 120 (e.g., in operative communication with an elongate electrode assembly 118), and an implanted second ferromagnetic portion 156 (e.g., an implanted second magnet). The first ferromagnetic portion 152 and the second ferromagnetic portion 156 are configured to be magnetically attracted to one another (e.g., one or both of the first ferromagnetic portion 152 and the second ferromagnetic portion 156 comprising a magnet having a static magnetic field) with the skin tissue 158 of the recipient therebetween.

The transmitter unit 128 comprises at least one first inductive communication coil 130 (e.g., a planar inductor coil having a plurality of turns) external to the recipient and the implanted receiver unit 132 comprises at least one second inductive communication coil 136 (e.g., a planar inductor coil having a plurality of turns) which form an transcutaneous inductive radio frequency (RF) communication link between the external component 142 and the internal component 144, across which the internal component 144 receives power and/or data signals from the external component 142. These inductive communication coils 130, 136 interact with one another via magnetic flux of one of the inductive communication coils 130, 136 passing through the other one of the inductive communication coils 130, 136. This interaction between the at least one first inductive communication coil 130 and the at least one second inductive communication coil 136 can be characterized by a coupling coefficient k (e.g., with 0 ≤ *k* ≤ 1), which can be understood to relate to (e.g., can be defined as) the fraction of magnetic flux produced by one of the coils 130, 136 that couples to the other of the coils 130, 136. The value of the coupling coefficient *k* is dependent on the geometries of the coils 130, 136, their relative distance from one another, and the offset between their axes 160, 162.

As schematically illustrated in FIG. 1B, the first ferromagnetic portion 152 is positioned relative to the at least one first inductive communication coil 130 and the second ferromagnetic portion 156 is positioned relative to the at least one second inductive communication coil 136 such that, when the external component 142 is placed on the recipient's skin 158, the at least one first inductive communication coil 130 is aligned with the at least one second inductive communication coil 136. For example, a centroid axis 160 of the first ferromagnetic portion 152 (e.g., an axis of symmetry of the shape of the first ferromagnetic portion 152; an axis of symmetry of a magnetic field produced by the first ferromagnetic portion 152) can be coincident with a center axis of the at least one first inductive communication coil 130, and a centroid axis 162 of the second ferromagnetic portion 156 (e.g., an axis of symmetry of the shape of the second ferromagnetic portion 156; an axis of symmetry of a magnetic field produced by the second ferromagnetic portion 156) can be coincident with a center axis of the at least one second inductive communication coil 136. When the external component 142 is sufficiently close to the internal component 144 such that the first ferromagnetic portion 152 and the second ferromagnetic portion 156 are magnetically attracted to one another, the centroid axis 160 of the first ferromagnetic portion 152 and the centroid axis 162 of the second ferromagnetic portion 156 are aligned with one another, thereby aligning the at least one first inductive communication coil 130 with the at least one second inductive communication coil 136.

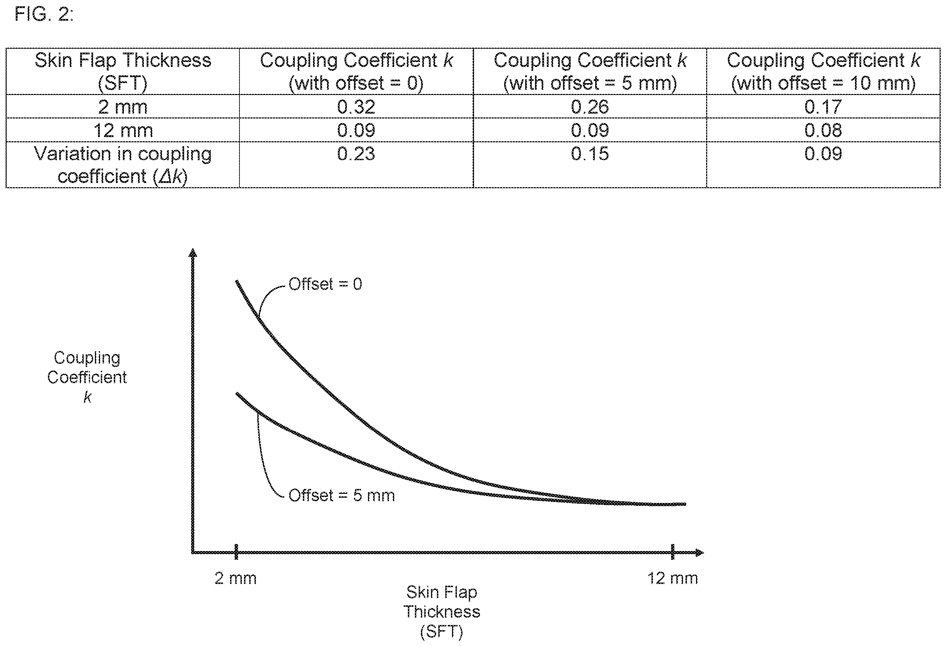

As illustrated by FIG. 2, the coupling coefficient k between the at least one first inductive communication coil 130 and the at least one second inductive communication coil 136 can vary significantly depending on the thickness of the recipient's skin 158 (e.g., "skin flap thickness" or "SFT") between the external component 142 and the internal component 144. Depending on the recipient, the SFT can be in a range of 2 millimeters to 12 millimeters, and the corresponding variation of the coupling coefficient *k* across this range of SFT can be large. For example, assuming an offset equal to zero between the center axes of the inductive communication coils 130, 136, the coupling coefficient *k* for SFT equal to 2 millimeters can be *k*_{SFT=2mm} = 0.32, while the coupling coefficient *k* for SFT equal to 12 millimeters can be *k*_{SFT=12mm} = 0.09.

In addition to differences of SFT among different recipients, the SFT can also change under various physiological situations (e.g., weight loss or gain by recipient; growth of the recipient). These changes of SFT present a challenge in tuning the RF communication link between the external component 142 and the internal component 144 for recipients having small values of SFT, since such changes of the SFT can result in large changes of the coupling coefficient k. For example, looking at the "Offset = 0" curve of FIG. 2, the large slope at low values of SFT (e.g., 2 mm) indicates that the coupling coefficient k undergoes significant change resulting from even small changes of SFT, while for larger values of SFT (e.g., 12 mm), the curve approaches an asymptote and the slope is smaller, indicating that the coupling coefficient k is less affected by changes of SFT. Such tuning challenges exist for transcutaneous systems utilizing a combined RF link (e.g., in which power and data are transmitted at the same time by modulating carrier waves) and for transcutaneous systems utilizing split RF links (e.g., in which power and data are each transmitted during separate and distinct time periods).

Rather than utilizing the largest possible coupling coefficient k for any given SFT (e.g., to maximize the power transfer efficiency between the external component 142 and the internal component 144), certain embodiments instead reduce the maximum value of the coupling coefficient k (e.g., to improve data integrity and/or RF link strength consistency) for a recipient having a small SFT value. In certain embodiments, a non-zero offset is introduced between the center axes of the inductive communication coils 130, 136. For example, looking at the "Offset = 5 mm" curve of FIG. 2, while the coupling coefficient *k* is smaller at low values of SFT (e.g., 2 mm), the slope of this portion of the curve is also smaller and the variation of the coupling coefficient k across the range of SFT is reduced. Therefore, changes of SFT have less effect on the coupling coefficient k when the inductive communication coils 130, 136 are offset from one another than when there is no offset. While the coil offset can be useful for low values of SFT, it has much less effect at high values of SFT (see, e.g., FIG. 2).

In certain embodiments, the introduction of an offset advantageously improves the overall efficiency of the RF communication link (e.g., by making the RF communication link easier to tune and/or making the strength of the RF link more consistent), improves data integrity, reduces fitting issues for clinicians, and/or gives recipients longer battery life. Furthermore, certain embodiments advantageously utilize the space within the cavity 210 in a beneficial way to improve the RF communication link. The offset can be introduced without modifications of the housing 150 or other portion of the external component 142 and/or the housing 154 or other portion of the internal component 144, thereby allowing certain embodiments described herein to be retrofitted with existing products to assist with tuning issues and/or to improve overall efficiency.

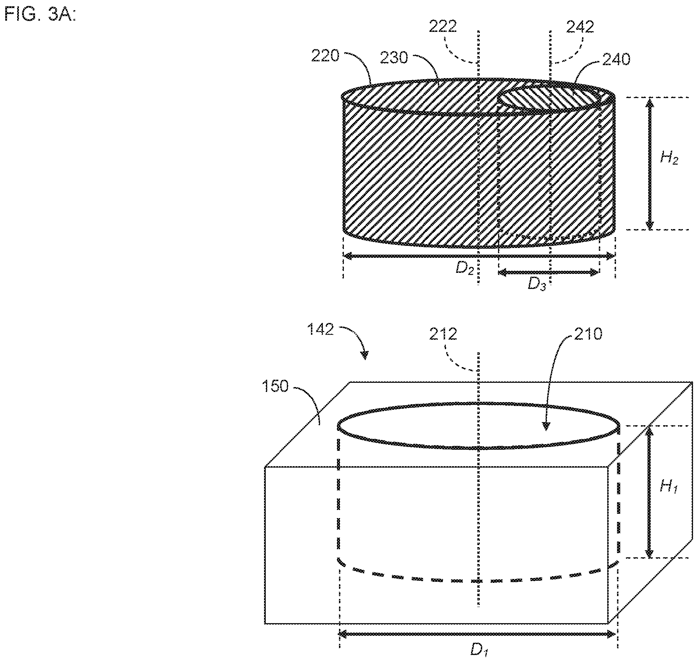

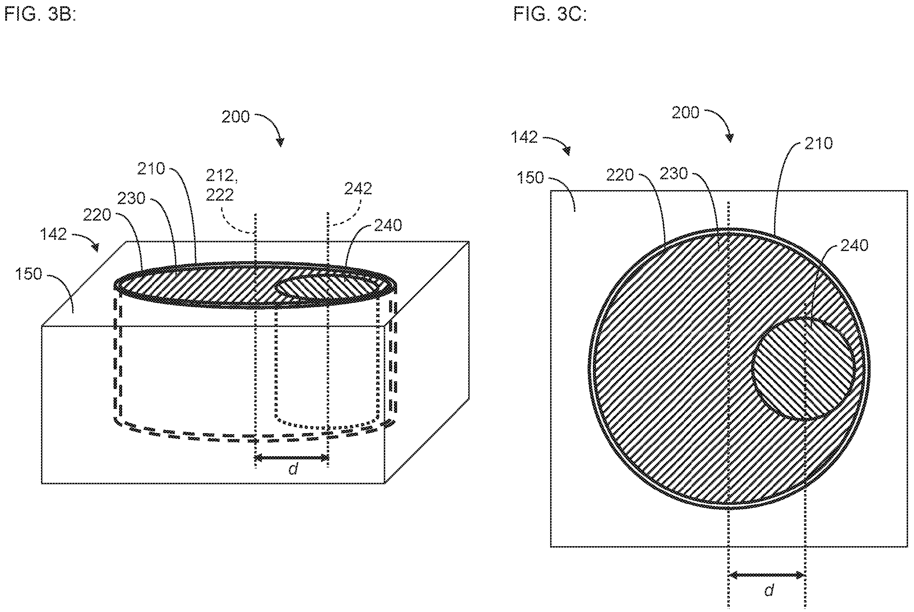

FIGs. 3A-3C schematically illustrate an example apparatus 200 in accordance with certain embodiments described herein. The apparatus 200 comprises a cavity 210 and a ferromagnet assembly 220 having a first centroidal axis 222. The ferromagnet assembly 220 is configured to be contained within the cavity 210. The ferromagnet assembly 220 comprises a first portion 230 comprising at least one non-ferromagnetic material and a second portion 240 comprising at least one ferromagnetic material. The second portion 240 has a second centroidal axis 242 that is offset from the first centroidal axis 222. FIG. 3A shows a schematic perspective view of the ferromagnet assembly 220 prior to being inserted within the cavity 210, FIG. 3B shows a schematic perspective view of the ferromagnet assembly 220 after being inserted within the cavity 210, and FIG. 3C shows a top view of the ferromagnet assembly 220 within the cavity 210.

In certain embodiments, the apparatus 200 is a component of an auditory prosthesis system, examples of which include but are not limited to: a cochlear implant system, a bone conduction implant system (e.g., active bone conduction system; passive bone conduction system, percutaneous bone conduction system; transcutaneous bone conduction system), a Direct Acoustic Cochlear Implant (DACI) system, a middle ear implant system, a middle ear transducer (MET) system, an electro-acoustic implant system, another type of auditory prosthesis system, and/or combinations or variations thereof.

In certain embodiments, the cavity 210 has a third centroidal axis 212 and is within the external component 142 of the auditory prosthesis system. In certain embodiments, the cavity 210 has a cylindrical shape with a cross-section (e.g., circular; elliptical; square; rectangular; polygonal; geometric; irregular; symmetric; non-symmetric) with straight, curved, or irregular sides in a plane perpendicular to a longitudinal axis of the cavity 210 and having a perimeter in the plane perpendicular to the longitudinal axis. For example, as schematically illustrated in FIGs. 3A-3C, the cavity 210 has a right circular cylindrical shape having a first diameter *D₁* and a first circumference *C₁* (*=* π*D₁*) in the plane perpendicular to the third centroidal axis 212 (e.g., the longitudinal axis of the cavity 210; an axis of symmetry of the cavity 210) and a first height *H₁* along the third centroidal axis 212. The first diameter *D₁* of certain embodiments is in a range of 6 millimeters to 14 millimeters, and the first height *H₁* of certain embodiments is in a range of 2 millimeters to 12 millimeters. Other shapes and/or sizes of the cavity 210 are also compatible with certain embodiments described herein.

While FIGs. 3A-3C schematically illustrate the cavity 210 as extending to a boundary (e.g., top surface) of a housing 150 of the external component 142, in certain other embodiments, the cavity 210 extends to another boundary (e.g., bottom surface; side surface) of the housing 150, extends to multiple boundaries (e.g., multiple surfaces) of the housing 150, or does not fully extend to any boundary (e.g., any surface) of the housing 150. In certain embodiments, the housing 150 comprises a plurality of portions (e.g., a main body and a lid) which are configured to be reversibly and repeatedly separated and rejoined (e.g., opened and closed) to allow access to the cavity 210 for inserting and/or removing the ferromagnet assembly 220 from the cavity 210. In certain such embodiments, the portions of the housing 150 are shaped to define the cavity 210 (e.g., a fraction of the cavity 210 is defined by a recess of a first housing portion and a remaining fraction of the cavity 210 is defined by a recess of a second housing portion).

In certain embodiments, the ferromagnet assembly 220 is configured to be contained within the cavity 210 such that the first centroidal axis 222 is coincident with the third centroidal axis 212. In certain embodiments, the ferromagnet assembly 220 has a cylindrical shape with a cross-section (e.g., circular; elliptical; square; rectangular; polygonal; geometric; irregular; symmetric; non-symmetric) with straight, curved, or irregular sides in a plane perpendicular to a longitudinal axis of the ferromagnet assembly 220 and having a perimeter in the plane perpendicular to the longitudinal axis. For example, as schematically illustrated in FIGs. 3A-3C, the ferromagnet assembly 220 has a right circular cylindrical shape having a second diameter *D₂* and a second circumference C2 (= π*D₂*) in the plane perpendicular to the first centroidal axis 222 (e.g., the longitudinal axis of the ferromagnet assembly 220; an axis of symmetry of the shape of the ferromagnet assembly 220) and a second height *H₂* along the first centroidal axis 222. The second diameter *D₂* is smaller than or equal to the first diameter *D₁*, the second circumference *C₂* is smaller than or equal to the first circumference *C₁,* and the second height *H₂* is smaller than or equal to the first height *H₁,* such that the ferromagnet assembly 220 is configured to fit within the cavity 210. The second diameter *D₂* of certain embodiments is in a range of 6 millimeters to 14 millimeters, and the second height *H₂* of certain embodiments is in a range of 2 millimeters to 12 millimeters. Other shapes and/or sizes of the ferromagnet assembly 220 are also compatible with certain embodiments described herein. For example, the cavity 210 and the ferromagnet assembly 220 can both have a non-symmetric shape such that the ferromagnet assembly 220 is configured to be contained within the cavity 210 in only one orientation.

In certain embodiments, the at least one non-ferromagnetic material of the first portion 230 of the ferromagnet assembly 220 comprises at least one of: plastic, epoxy, ceramic, titanium, aluminum, and a non-ferromagnetic metal. In certain embodiments, the at least one ferromagnetic material of the second portion 240 of the ferromagnet assembly 220 comprises at least one of: iron, nickel, cobalt, and steel. The second portion 240 of certain embodiments comprises a permanent magnet having an external static magnetic field, while in certain other embodiments, the second portion 240 does not comprise a permanent magnet but is configured to be magnetically attracted to a ferromagnet portion of a magnet beneath the skin of the recipient. Thus, in certain embodiment, the ferromagnet assembly 220 does not comprise a magnet, but does comprise a ferromagnetic material. The ferromagnet assembly 220 of certain embodiments is a unitary member such that the first portion 230 and the second portion 240 cannot be easily separated from one another without damaging the ferromagnet assembly 220, while in certain other embodiments, the first portion 230 and the second portion 240 are configured to be reversibly and repeatedly separated from and rejoined to one another without damaging the ferromagnet assembly 220.

While FIGs. 3A-3C schematically illustrates the second portion 240 as extending to a boundary (e.g., top surface) of the ferromagnet assembly 220, in certain other embodiments, the second portion 240 extends to another boundary (e.g., bottom surface; side surface) of the ferromagnet assembly 220, extends to multiple boundaries (e.g., multiple surfaces) of the ferromagnet assembly 220, or does not fully extend to any boundary (e.g., surface) of the ferromagnet assembly 220. In certain embodiments, the first portion 230 of the ferromagnet assembly 220 comprises a plurality of sub-portions (e.g., a main body and a lid) which are configured to be reversibly and repeatedly separated and rejoined (e.g., opened and closed) to allow access to an internal region of the ferromagnet assembly 220 for inserting and/or removing the second portion 240 from the ferromagnet assembly 220.

In certain embodiments, the second portion 240 has a cylindrical shape with a cross-section (e.g., circular; square; rectangular; polygonal; geometric; irregular; symmetric; non-symmetric) with straight, curved, or irregular sides in a plane perpendicular to a longitudinal axis of the second portion 240 and having a perimeter in the plane perpendicular to the longitudinal axis. For example, as schematically illustrated in FIGs. 3A-3C, the second portion 240 has a right circular cylindrical shape having a third diameter *D₃* and a third circumference *C₃* (= π*D₃*) in the plane perpendicular to the second centroidal axis 242 (e.g., the longitudinal axis of the second portion 240; an axis of symmetry of the shape of the second portion 240; an axis of symmetry of a magnetic field produced by the second portion 240; a center axis of a magnetic field produced by the second portion 240) and a third height *H₃* along the second centroidal axis 242. The third diameter *D₃* is smaller than the second diameter *D₂,* the third circumference *C₃* is smaller than the second circumference *C₂,* and the third height *H₃* is smaller than or equal to the second height *H₂.* The third diameter *D₃* of certain embodiments is in a range of 2 millimeters to 12 millimeters, and the third height *H₃* of certain embodiments is in a range of 2 millimeters to 12 millimeters. Other shapes and/or sizes of the second portion 240 are also compatible with certain embodiments described herein.

As schematically illustrated by FIGs. 3A-3C, the second centroidal axis 242 of certain embodiments is parallel to the first centroidal axis 222, while in certain other embodiments, the second centroidal axis 242 is non-parallel to the first centroidal axis 222. In certain embodiments, the second centroidal axis 242 is offset from the first centroidal axis 222 by a distance *d*, which, in certain embodiments, is in a range of 1 millimeter to 6 millimeters.

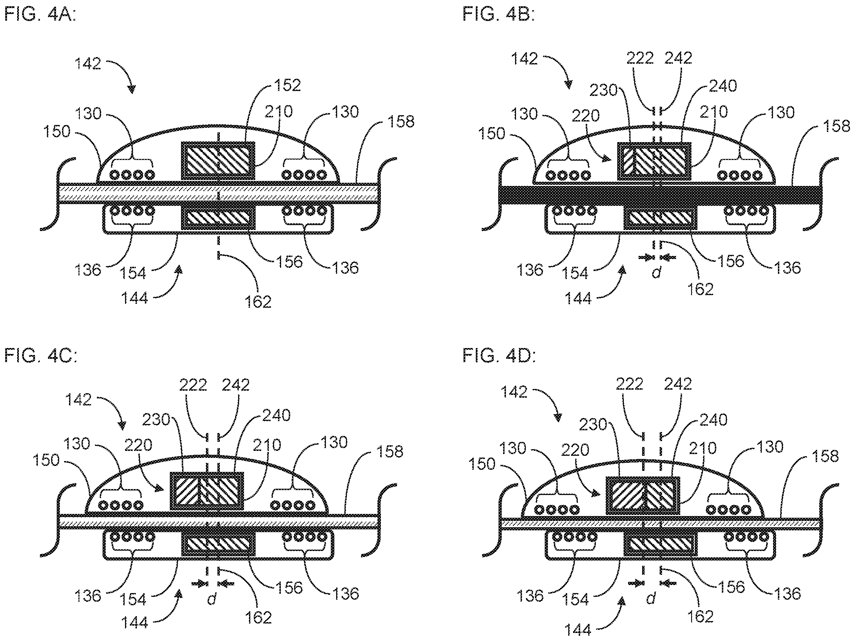

FIG. 4A schematically illustrates an example apparatus 200 utilized with a first skin flap thickness (also schematically illustrated in FIG. 1B). FIGs. 4B-4D schematically illustrate an example apparatus 200 utilized with different skin flap thicknesses in accordance with certain embodiments described herein. The apparatus 200 comprises a housing (e.g., housing 150 of the external component 142 of an auditory prosthesis) configured to be placed over a portion of skin 158 of a recipient, the portion of skin 158 overlaying an implanted device (e.g., an internal component 144 of an auditory prosthesis). The implanted device comprises an internal inductive communication coil (e.g., at least one internal inductive communication coil 136) and an internal ferromagnetic material (e.g., the implanted second ferromagnetic portion 156 of the internal component 144). The apparatus 200 further comprises an external inductive communication coil (e.g., at least one first inductive communication coil 130) within the housing, the external inductive communication coil configured to be in inductive communication with the internal inductive communication coil. The apparatus 200 further comprises an external ferromagnet (e.g., the ferromagnet assembly 220) contained with a cavity 210 of the housing and substantially filling the cavity 210. The external ferromagnet comprises a ferromagnetic portion (e.g., the second portion 240 of the ferromagnet assembly 220) and a non-ferromagnetic portion (e.g., the first portion 230 of the ferromagnet assembly 220), the ferromagnetic portion configured to be magnetically attracted to the internal ferromagnetic material. The cavity 210 has a center (e.g., along the third centroidal axis 212) that defines a line with a center of the external inductive communication coil, and the ferromagnetic portion of the external ferromagnet has a center (e.g., along the second centroidal axis 242) that is offset from the line defined by the center of the cavity and the center of the external inductive communication coil.

In certain embodiments, as schematically illustrated by FIGs. 4A-4D, the internal ferromagnetic portion 156 is concentric with the internal inductive communication coil 136 (e.g., the center of the internal ferromagnetic portion 156 is aligned with a center of the internal inductive communication coil 136 in a direction perpendicular to a plane of the internal inductive communication coil 136) and the cavity 210 is concentric with the external inductive communication coil 130 (e.g., the center of the cavity 210 is aligned with a center of the external inductive communication coil 130 in a direction perpendicular to a plane of the external inductive communication coil 130). As used herein, the term "concentric" refers to the relative positions of the centers of two or more components, and does not refer to any particular shapes of these components. For example, the cavity 210 and the external inductive communication coil 130 can be concentric with one another without either the cavity 210 or the external inductive communication coil 130 having a circular shape.

In certain embodiments, the internal ferromagnetic portion 156 and the second portion 240 of the ferromagnet assembly 220 are configured to be magnetically attracted to one another (e.g., one or both of the internal ferromagnetic portion 156 and the second portion 240 comprising a magnet having a static magnetic field) with the skin tissue 158 of the recipient therebetween. In certain embodiments, the implanted second ferromagnetic portion 156 is encircled by the at least one second inductive communication coil 136 and the ferromagnet assembly 220 (including the second portion 240) is encircled by the at least one first inductive communication coil 130. In addition, as schematically illustrated by FIGs. 4B-4D, while the ferromagnet assembly 220 is concentric with the at least one first inductive communication coil 130, the second portion 240 of the ferromagnet assembly 220 is not concentric with the at least one first inductive communication coil 130 (e.g., the first centroid axis 222 of the ferromagnet assembly 220 is offset from the second centroid axis 242 of the second portion 240).

In certain embodiments, the ferromagnet assembly 220 can be selected based, at least in part on the skin flap thickness, to provide a predetermined offset d between the at least one first inductive communication coil 130 of the external component 142 and the at least one second inductive communication coil 136 of the implanted component 144. For example, as schematically illustrated by FIGs. 4A-4D, the skin flap thickness of FIG. 4A (e.g., 12 mm) is larger than the skin flap thickness of FIG. 4B (e.g., 9 mm), which is larger than the skin flap thickness of FIG. 4C (e.g., 6 mm), which is larger than the skin flap thickness of FIG. 4D (e.g., 3 mm). For the skin flap thickness of FIG. 4A, the ferromagnetic material substantially fills the cavity 210 such that the magnetic attraction between the ferromagnetic material of the external component 142 and the implanted ferromagnetic portion 156 produces an offset *d* = 0 between the at least one first inductive communication coil 130 of the external component 142 and the at least one second inductive communication coil 136 of the implanted component 144.

As schematically illustrated by FIGs. 4B-4D, for smaller skin flap thicknesses, the magnetic attraction between the second portion 240 of the ferromagnet assembly 220 and the implanted ferromagnetic portion 156 substantially aligns the second portion 240 (e.g., the second centroid axis 242) with the implanted ferromagnetic portion 156 (e.g., the centroid axis 162). In certain embodiments, the cavity 210 and the ferromagnet assembly 220 are concentric with the at least one first inductive communication coil 130 and the second portion 240 of the ferromagnet assembly 220 is not concentric with the at least one first inductive communication coil 130, and the offset *d* between the first centroid axis 222 of the ferromagnet assembly 220 and the second centroid axis 242 of the second portion 240 results in the same offset *d* between the at least one first inductive communication coil 130 and the at least one second inductive communication coil 136. With smaller skin flap thicknesses, a larger offset *d* can be selected. In certain embodiments, the magnetic strength of the ferromagnet assembly 220 and the offset *d* between the at least one first inductive communication coil 130 and the at least one second inductive communication coil 136 are both dependent on the skin flap thickness, so for a known skin flap thickness, a ferromagnet assembly 220 providing an appropriate magnetic strength and an appropriate offset can be selected.

The examples of FIGs. 4A-4D discussed herein have the cavity 210 concentric with the at least one first inductive communication coil 130 and the implanted ferromagnetic portion 156 concentric with the at least one second inductive communication coil 136. Certain other embodiments utilize non-concentric configurations but with the displacement (e.g., magnitude and direction) between the centers of the cavity 210 and the at least one first inductive communication coil 130 substantially equal to the displacement between the centers of the implanted ferromagnetic portion 156 and the at least one second inductive communication coil 136. As with the examples of FIGs. 4A-4D, the offset *d* is produced by using a second portion 240 of the ferromagnet assembly 220 that has a center that is laterally displaced (e.g., in a direction parallel to the skin 158) from a center of the cavity 210 by an amount that is selected based on a thickness of the portion of skin 158 between the housing 150 of the external component 142 and the implanted component 144.

In certain embodiments, in addition to having the position of the second portion 240 within the cavity 210 selected, based on a thickness of the portion of skin 158, to produce a predetermined offset *d* between the at least one first inductive communication coil 130 and the at least one second inductive communication coil 136, the size of the second portion 240 of the ferromagnet assembly 220 is selected to provide a predetermined amount of magnetic attraction between the external component 142 and the internal component 144 (e.g., a sufficient amount of magnetic attraction to maintain the position of the external component 142 on the recipient). The second portion 240 of certain embodiments has one or more lateral dimensions (e.g., in a direction parallel to the skin 158) that are smaller than corresponding lateral dimensions of the implanted ferromagnetic portion 156. For example, the implanted ferromagnetic portion 156 can have a first perimeter *P₁* in a plane parallel to the portion of skin 158 and the second portion 240 can have a second perimeter *P₂* in a plane parallel to the portion of skin 158 (e.g., the third circumference *C₃* in the plane perpendicular to the second centroidal axis 242) that is smaller than the first perimeter *P₁.* In certain embodiments the first perimeter *P₁* is in a range of 60 millimeters to 72 millimeters, and the second perimeter *P₂* (e.g., third circumference *C₃*) is in a range of 6 millimeters to 70 millimeters. In certain embodiments, the perimeter (e.g., outer circumference) of the at least one first inductive communication coil 130 is in a range of 60 millimeters to 110 millimeters and the perimeter (e.g., outer circumference) of the at least one second inductive communication coil 136 is in a range of 60 millimeters to 110 millimeters. In addition, in certain embodiments, a direction of the offset can be selected (e.g., using a cavity 210 and ferromagnet assembly 220 that are "keyed" by a non-symmetric feature to have the offset occur in a predetermined lateral direction. For example, the second portion 240 can be positioned closer to a region of the external component 142 expected to experience larger forces that may inadvertently remove or displace the external component 142 relative to the recipient during operation (e.g., a region near wires or leads connected to the external component 142).

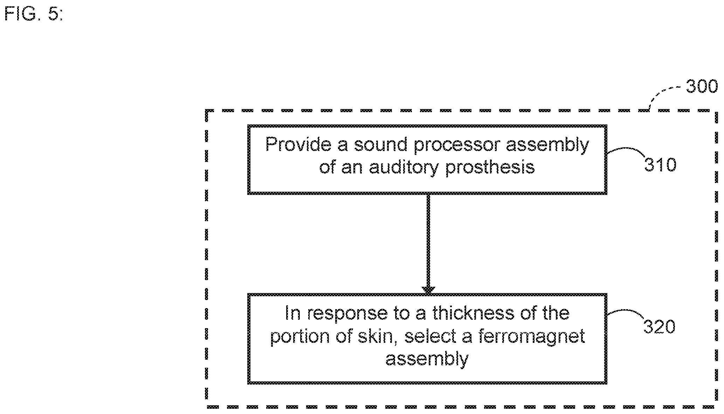

FIG. 5 is a flow diagram of an example method 300 in accordance with certain embodiments described herein. In an operational block 310, the method 300 comprises providing a sound processor assembly (e.g., external component 142) of an auditory prosthesis. The assembly is configured to be placed over a portion of skin 158 of a recipient overlaying an implanted portion (e.g., internal component 144) of the auditory prosthesis. The sound processor assembly comprises at least one external inductive communication coil (e.g., at least one first inductive communication coil 130) configured to be in inductive communication with at least one internal inductive communication coil (e.g., at least one second inductive communication coil 136) of the implanted portion of the auditory prosthesis. The sound processor assembly further comprises a cavity 210 configured to contain a ferromagnet assembly 220.

In an operational block 320, the method 300 further comprises, in response to a thickness of the portion of skin 158, selecting a ferromagnet assembly 200 from two or more ferromagnet assemblies 220 configured to be contained within the cavity 210. Each of the two or more ferromagnet assemblies 220 is configured to, upon being contained within the cavity 210 and the sound processor assembly being placed over the portion of skin 158, displace the at least one external inductive communication coil relative to the at least one internal inductive communication coil by a corresponding offset in a direction parallel to the portion of skin 158 while providing sufficient magnetic force to retain the sound processor assembly over the portion of skin 158.

In certain embodiments, each of the two or more ferromagnet assemblies 220 has a corresponding ferromagnetic portion (e.g., second portion 240; a ferromagnet; a non-magnetic ferromagnetic portion) and a corresponding non-magnetic portion (e.g., first portion 230), with the ferromagnetic portion having a corresponding offset relative to a center of the at least one external inductive communication coil. In certain embodiments, the method 300 further comprises inserting the selected ferromagnet assembly 220 into the cavity 210. For example, a clinician can evaluate the skin flap thickness of the recipient, can select (e.g., prescribe), based at least in part on the skin flap thickness, a ferromagnet assembly 220 having a second portion 240 that is configured to provide a predetermined offset appropriate for the recipient's skin flap thickness, and either the clinician or the recipient can insert the selected ferromagnet assembly 220 in the cavity 210. At lower skin flap thicknesses, ferromagnetic assemblies 220 with weaker magnetic force can be prescribed, and at higher skin flap thicknesses, ferromagnetic assemblies 220 with stronger magnetic force can be prescribed (e.g., to ensure adequate retention of the at least one external inductive communication coil on the recipient). In certain embodiments, the selected ferromagnet assembly 220 also displaces the at least one external inductive communication coil to reduce a coupling coefficient between the at least one external inductive communication coil and the at least one internal inductive communication coil as compared to a coupling coefficient between the at least one external inductive communication coil and the at least one internal inductive communication coil with no offset in the direction parallel to the portion of skin 158.

It is to be appreciated that the embodiments disclosed herein are not mutually exclusive and may be combined with one another in various arrangements.

The invention described and claimed herein is not to be limited in scope by the specific example embodiments herein disclosed, since these embodiments are intended as illustrations, and not limitations, of several aspects of the invention. Indeed, various modifications of the invention in form and detail, in addition to those shown and described herein, will become apparent to those skilled in the art from the foregoing description. Such modifications are also intended to fall within the scope of the claims.

## Claims

1. An apparatus (100) comprising:
a cavity (210); and
a ferromagnet assembly (220) having a first centroidal axis (222), the ferromagnet assembly (220) configured to be contained within the cavity (210) the ferromagnet assembly (220) comprising:
a first portion (230) comprising at least one non-ferromagnetic material; and
a second portion (240) comprising at least one ferromagnetic material, the second portion (240) having a second centroidal axis (242) that is offset from the first centroidal axis (222), **characterised in that** the ferromagnetic assembly (220) is configured to be removable from the cavity (210).

2. The apparatus (100) of claim 1, wherein the second centroidal axis (242) is parallel to the first centroidal axis (222).

3. The apparatus (100) of claim 1 or claim 2, wherein the cavity (210) has a third centroidal axis (212) and the ferromagnetic assembly (220) is configured to be contained within the cavity (210) such that the first centroidal axis (222) is coincident with the third centroidal axis (212).

4. The apparatus (100) of claim 3, wherein the cavity (210) has a right circular cylindrical shape having a first diameter, the third centroidal axis (212) is an axis of symmetry of the cavity (210), the ferromagnet assembly (220) has a right circular cylindrical shape having a second diameter, the second diameter smaller than or equal to the first diameter such that the ferromagnet assembly (220) fits within the cavity (210), and the first centroidal axis (222) is an axis of symmetry of the ferromagnet assembly (220).

5. The apparatus (100) of claim 4, wherein the cavity (210) has a first height, and the ferromagnet assembly (220) has a second height smaller than or equal to the first height such that the ferromagnet assembly (220) is configured to fit within the cavity (210).

6. The apparatus (100) of claim 4, wherein the second portion (240) has a right circular cylindrical shape having a third diameter smaller than the second diameter and the second centroidal axis (242) is an axis of symmetry of the second portion (240).

7. The apparatus (100) of claim 6, wherein the second portion (240) has a height substantially equal to the second height.

8. The apparatus (100) of any of claims 4 to 6, wherein the first diameter is in a range of 6 millimeters to 14 millimeters, the first height is in a range of 2 millimeters to 12 millimeters, the second diameter is in a range of 6 millimeters to 14 millimeters, the second height is in a range of 2 millimeters to 12 millimeters, and the third diameter is in a range of 2 millimeters to 12 millimeters.

9. The apparatus (100) of any preceding claim, wherein the at least one non-ferromagnetic material comprises at least one of: plastic, epoxy, ceramic, titanium, aluminum, and a non-ferromagnetic metal.

10. The apparatus (100) of any preceding claim, wherein the at least one ferromagnetic material has an external magnetic field.

11. The apparatus (100) of any preceding claim, wherein the cavity (210) and the ferromagnet assembly (220) both have a non-symmetric shape such that the ferromagnet assembly (220) is configured to be contained within the cavity (210) in only one orientation.

12. The apparatus (100) of any preceding claim, wherein the ferromagnet assembly (220) is configured to substantially fill the cavity (210).

13. The apparatus (100) of any preceding claim, further comprising a housing (150) defining the cavity (210), the housing (150) comprising a plurality of portions configured to be reversibly and repeatedly separated and rejoined to allow access to the cavity (210) for inserting and/or removing the ferromagnet assembly (220) from the cavity (210).

14. The apparatus (100) of claim 13, wherein the plurality of portions of the housing (150) comprise a main body and a lid.

15. The apparatus (100) of claim 13 or claim 14, wherein the plurality of portions of the housing (150) are shaped to define the cavity (210).

## Patentansprüche

1. Einrichtung (100), umfassend:
einen Hohlraum (210); und
eine ferromagnetische Anordnung (220) mit einer ersten Schwerpunktsachse (222), wobei die ferromagnetische Anordnung (220) so konfiguriert ist, dass sie in dem Hohlraum (210) enthalten ist, wobei die ferromagnetische Anordnung (220)
Folgendes umfasst:
einen ersten Abschnitt (230), umfassend mindestens ein nichtferromagnetisches Material; und
einen zweiten Abschnitt (240), umfassend mindestens ein ferromagnetisches Material,
wobei der zweite Abschnitt (240) eine zweite Schwerpunktsachse (242) aufweist, die gegenüber der ersten Schwerpunktsachse (222) versetzt ist, **dadurch gekennzeichnet, dass** die ferromagnetische Anordnung (220) so konfiguriert ist, dass sie aus dem Hohlraum (210) entfernbar ist.

2. Einrichtung (100) nach Anspruch 1, wobei die zweite Schwerpunktsachse (242) parallel zur ersten Schwerpunktsachse (222) verläuft.

3. Einrichtung (100) nach Anspruch 1 oder Anspruch 2, wobei der Hohlraum (210) eine dritte Schwerpunktsachse (212) aufweist und die ferromagnetische Anordnung (220) so konfiguriert ist, dass sie innerhalb des Hohlraums (210) enthalten ist, so dass die erste Schwerpunktsachse (222) mit der dritten Schwerpunktsachse (212) zusammenfällt.

4. Einrichtung (100) nach Anspruch 3, wobei der Hohlraum (210) eine Form eines rechtszirkularen Zylinders mit einem ersten Durchmesser aufweist, die dritte Schwerpunktsachse (212) eine Symmetrieachse des Hohlraums (210) ist, die ferromagnetische Anordnung (220) eine Form eines rechtszirkularen Zylinders mit einem zweiten Durchmesser aufweist, wobei der zweite Durchmesser kleiner oder gleich dem ersten Durchmesser ist, so dass die ferromagnetische Anordnung (220) in den Hohlraum (210) passt, und die erste Schwerpunktsachse (222) eine Symmetrieachse der ferromagnetischen Anordnung (220) ist.

5. Einrichtung (100) nach Anspruch 4, wobei der Hohlraum (210) eine erste Höhe aufweist, und die ferromagnetische Anordnung (220) eine zweite Höhe aufweist, die kleiner oder gleich der ersten Höhe ist, so dass die ferromagnetische Anordnung (220) so konfiguriert ist, dass sie in den Hohlraum (210) passt.

6. Einrichtung (100) nach Anspruch 4, wobei der zweite Abschnitt (240) eine Form eines rechtszirkularen Zylinders mit einem dritten Durchmesser aufweist, der kleiner ist als der zweite Durchmesser, und die zweite Schwerpunktsachse (242) eine Symmetrieachse des zweiten Abschnitts (240) ist.

7. Einrichtung (100) nach Anspruch 6, wobei der zweite Abschnitt (240) eine Höhe aufweist, die im Wesentlichen gleich der zweiten Höhe ist.

8. Einrichtung (100) nach einem der Ansprüche 4 bis 6, wobei der erste Durchmesser in einem Bereich von 6 Millimetern bis 14 Millimetern liegt, die erste Höhe in einem Bereich von 2 Millimetern bis 12 Millimetern liegt, der zweite Durchmesser in einem Bereich von 6 Millimetern bis 14 Millimetern liegt, die zweite Höhe in einem Bereich von 2 Millimetern bis 12 Millimetern liegt, und der dritte Durchmesser in einem Bereich von 2 Millimetern bis 12 Millimetern liegt.

9. Einrichtung (100) nach einem vorstehenden Anspruch, wobei das mindestens eine nicht-ferromagnetische Material mindestens eines von Folgendem umfasst: Kunststoff, Epoxid, Keramik, Titan, Aluminium und ein nichtferromagnetisches Metall.

10. Einrichtung (100) nach einem vorstehenden Anspruch, wobei das mindestens eine ferromagnetische Material ein externes Magnetfeld aufweist.

11. Einrichtung (100) nach einem vorstehenden Anspruch, wobei der Hohlraum (210) und die ferromagnetische Anordnung (220) beide eine nichtsymmetrische Form aufweisen, so dass die ferromagnetische Anordnung (220) so konfiguriert ist, dass sie nur in einer Ausrichtung innerhalb des Hohlraums (210) enthalten ist.

12. Einrichtung (100) nach einem vorstehenden Anspruch, wobei die ferromagnetische Anordnung (220) so konfiguriert ist, dass sie den Hohlraum (210) im Wesentlichen ausfüllt.

13. Einrichtung (100) nach einem vorstehenden Anspruch, weiter umfassend ein Gehäuse (150), das den Hohlraum (210) definiert, wobei das Gehäuse (150) eine Vielzahl von Abschnitten umfasst, die so konfiguriert sind, dass sie reversibel und wiederholt getrennt und wieder verbunden werden, um einen Zugang zum Hohlraum (210) zu ermöglichen, um die ferromagnetische Anordnung (220) in den Hohlraum (210) einzuführen und/oder aus diesem zu entfernen.

14. Einrichtung (100) nach Anspruch 13, wobei die Vielzahl von Abschnitten des Gehäuses (150) einen Hauptkörper und einen Deckel umfasst.

15. Einrichtung (100) nach Anspruch 13 oder Anspruch 14, wobei die Vielzahl von Abschnitten des Gehäuses (150) so geformt sind, dass sie den Hohlraum (210) definieren.

## Revendications

1. Appareil (100) comprenant :
une cavité (210) ; et
un ensemble ferromagnétique (220) présentant un premier axe centroïdal (222), l'ensemble ferromagnétique (220) étant configuré pour être contenu à l'intérieur de la cavité (210), l'ensemble ferromagnétique (220) comprenant :
une première portion (230) comprenant au moins un matériau non ferromagnétique ; et
une deuxième portion (240) comprenant au moins un matériau ferromagnétique,
la deuxième portion (240) présentant un deuxième axe centroïdal (242) qui est décalé par rapport au premier axe centroïdal (222), **caractérisé en ce que** l'ensemble ferromagnétique (220) est configuré pour être retiré de la cavité (210).

2. Appareil (100) selon la revendication 1, dans lequel le deuxième axe centroïdal (242) est parallèle au premier axe centroïdal (222).

3. Appareil (100) selon la revendication 1 ou la revendication 2, dans lequel la cavité (210) présente un troisième axe centroïdal (212) et l'ensemble ferromagnétique (220) est configuré pour être contenu à l'intérieur de la cavité (210) de telle sorte que le premier axe centroïdal (222) coïncide avec le troisième axe centroïdal (212).

4. Appareil (100) selon la revendication 3, dans lequel la cavité (210) présente une forme cylindrique circulaire droite présentant un premier diamètre, le troisième axe centroïdal (212) est un axe de symétrie de la cavité (210), l'ensemble ferromagnétique (220) présente une forme cylindrique circulaire droite présentant un deuxième diamètre, le deuxième diamètre étant inférieur ou égal au premier diamètre de telle sorte que l'ensemble ferromagnétique (220) s'insère dans la cavité (210), et le premier axe centroïdal (222) est un axe de symétrie de l'ensemble ferromagnétique (220).

5. Appareil (100) selon la revendication 4, dans lequel la cavité (210) présente une première hauteur, et l'ensemble ferromagnétique (220) présente une deuxième hauteur inférieure ou égale à la première hauteur de telle sorte que l'ensemble ferromagnétique (220) est configuré pour s'insérer dans la cavité (210).

6. Appareil (100) selon la revendication 4, dans lequel la deuxième portion (240) présente une forme cylindrique circulaire droite présentant un troisième diamètre inférieur au deuxième diamètre et le deuxième axe centroïdal (242) est un axe de symétrie de la deuxième portion (240).

7. Appareil (100) selon la revendication 6, dans lequel la deuxième portion (240) présente une hauteur sensiblement égale à la deuxième hauteur.

8. Appareil (100) selon l'une quelconque des revendications 4 à 6, dans lequel le premier diamètre est dans une plage de 6 millimètres à 14 millimètres, la première hauteur est dans une plage de 2 millimètres à 12 millimètres, le deuxième diamètre est dans une plage de 6 millimètres à 14 millimètres, la deuxième hauteur est dans une plage de 2 millimètres à 12 millimètres, et le troisième diamètre est dans une plage de 2 millimètres à 12 millimètres.

9. Appareil (100) selon une quelconque revendication précédente, dans lequel l'au moins un matériau non ferromagnétique comprend au moins l'un parmi : plastique, époxy, céramique, titane, aluminium et métal non ferromagnétique.

10. Appareil (100) selon une quelconque revendication précédente, dans lequel l'au moins un matériau ferromagnétique présente un champ magnétique externe.

11. Appareil (100) selon une quelconque revendication précédente, dans lequel la cavité (210) et l'ensemble ferromagnétique (220) présentent tous les deux une forme non symétrique de telle sorte que l'ensemble ferromagnétique (220) est configuré pour être contenu à l'intérieur de la cavité (210) dans une seule orientation.

12. Appareil (100) selon une quelconque revendication précédente, dans lequel l'ensemble ferromagnétique (220) est configuré pour remplir sensiblement la cavité (210).

13. Appareil (100) selon une quelconque revendication précédente, comprenant en outre un logement (150) définissant la cavité (210), le logement (150) comprenant une pluralité de portions configurées pour être séparées et réunies de façon réversible et de façon répétée pour permettre l'accès à la cavité (210) afin d'insérer et/ou de retirer l'ensemble ferromagnétique (220) de la cavité (210).

14. Appareil (100) selon la revendication 13, dans lequel la pluralité de portions du logement (150) comprennent un corps principal et un couvercle.

15. Appareil (100) selon la revendication 13 ou la revendication 14, dans lequel la pluralité de portions du logement (150) sont mises en forme pour définir la cavité (210).
